# EUROPEAN PATENT APPLICATION

(11) **EP 3 384 861 A1**
(43) Date of publication of application: **10.10.2018**
(21) Application number: 16871044.0
(22) Date of filing: 30.11.2016
(51) Int. Cl.: A61B 17/34

(54) **SURGICAL TROCAR**

(30) Priority: 01.12.2015 KR 20150169800
(71) Applicant: THE INDUSTRY & ACADEMIC COOPERATION IN CHUNGNAM NATIONAL UNIVERSITY (IAC), Daejeon 34134 (KR); Medi Tulip Co., Ltd., Cheongju-si, Chungcheongbuk-do 28160 (KR)
(72) Inventor: KANG, Min Woong, Daejeon 34896 (KR)
(74) Representative: Vossius & Partner Patentanwälte Rechtsanwälte mbB
(86) International application number: PCT/KR2016/013996
(87) International publication number: WO 2017/095148

(57) **Abstract**

The present invention provides a surgical trocar comprising a connection unit, which: connects a first trocar body and a second trocar body such that the second trocar body can vertically move relative to the first trocar body when a trocar unit is inserted into and drawn out of a trocar body unit having the first trocar body and the second trocar body, such that when an external force is not applied, a state of being unfolded in the radial direction of the first trocar body is maintained; and has an extension member extending in the longitudinal direction of the first trocar body when the trocar unit is inserted into the trocar body unit such that the second trocar body moves relative to the first trocar body.

## Description

### [Technical Field]

The present invention relates to a surgical trocar and, more particularly, to a surgical trocar which has a simple structure and is easy to use while stably supporting a lower portion thereof inside the human body without any additional mechanical equipment.

### [Background Art]

Generally, typical laparotomy has a problem in that recovery of a patient is slowed down due to a large incision and large blood loss during surgery and a large scar remaining after surgery can interfere with the patient's later life.

Recently, new surgical techniques such as minimally invasive surgery (MIS) have been developed to overcome the disadvantages of laparotomy.

Minimally invasive surgery is a set of techniques that allow a surgeon to operate through minimized incisions using specially-designed elongated instruments and requires a trocar for securing free advance and retreat of the instruments.

A typical trocar is provided at a tip thereof with a balloon-type support which is inflatable to secure the trocar disposed through the abdominal wall or the chest wall to the abdominal wall or the chest wall. However, such a balloon-type support can easily burst and requires separate mechanical equipment for injecting air into the support.

### [Disclosure]

### [Technical Problem]

It is an aspect of the present invention to provide a surgical trocar which has a simple structure and is easy to use while supporting a lower portion thereof inside the human body without any additional mechanical equipment.

### [Technical Solution]

In accordance with an aspect of the present invention, a surgical trocar includes: a trocar cannula capable of being disposed through human skin tissue; and a trocar rod inserted into and withdrawn from the trocar cannula, wherein the trocar cannula includes: a first cannula body having a hollow shape; a cannula head disposed at an upper side of the first cannula body; a second cannula body disposed below the first cannula body to be separated a predetermined distance from the first cannula body; and a connection unit connecting the first cannula body to the second cannula body such that the second cannula body is vertically moved relative to the first cannula body when the trocar rod is inserted into or withdrawn from the trocar cannula, wherein the connection unit includes an extension member which is kept spread in a radial direction of the first cannula body when not subjected to external force and is stretched in a longitudinal direction of the first cannula body when the trocar rod is inserted into the trocar cannula such that the second cannula body is moved relative to the first cannula body.

### [Advantageous Effects]

The present invention provides a surgical trocar which includes an extension member disposed at a lower portion of a trocar cannula, wherein the extension member is deformed by external force and is returned to an original shape thereof when external force is removed, such that the insertion radius of the trocar cannula can be minimized in the process of inserting the lower portion of the trocar cannula into the human body, thereby allowing the human skin tissue to be minimally incised. In addition, after the lower portion of the trocar cannula is inserted into the human body, the extension member is spread in a radial direction of the trocar cannula, thereby stably securing the trocar cannula inside the human body.

In addition, the surgical trocar according the present invention includes a locking unit which can keep the extension member stretched in the longitudinal direction of the trocar cannulawhen a user inserts a trocar rod into the trocar cannula, such that the user can put the surgical trocar into the human body with the extension member stretched in the longitudinal direction of the trocar cannula, thereby eliminating a need for the user to continue to hold the trocar rod and the trocar cannula at the same time.

Further, since the locking unit allows the extension member to remain stretched in the longitudinal direction of the trocar cannula, it is possible to solve a problem caused by the extension member which would otherwise be spread in the radial direction of the trocar cannula, that is, a difficulty in putting the surgical trocar into the human body.

### [Description of Drawings]

FIG. 1 is a sectional view of main components of a surgical trocar according to one embodiment of the present invention.
FIG. 2 is a sectional view illustrating a state in which a trocar rod of the surgical trocar shown in FIG. 1 is inserted into a trocar cannula such that the trocar cannula is stretched.
FIG. 3 is a sectional view illustrating a state in which the trocar cannula is secured to the human skin tissue after the trocar rod is removed from the trocar cannula in the state of FIG. 2.
FIG. 4 is a view illustrating coupling between an upper portion of the trocar rod of the surgical trocar shown in FIG. 1 and an inner housing of a cannula head.
FIG. 5 is a view illustrating coupling between a coupling protrusion of the trocar rod shown in FIG. 4 and a protrusion coupling portion of the inner housing.

### [Best Mode]

Exemplary embodiments of the present invention will be described with reference to the accompanying drawings. It should be noted that like components will be denoted by like reference numerals throughout the specification and the accompanying drawings. In addition, descriptions of details apparent to those skilled in the art will be omitted for clarity.

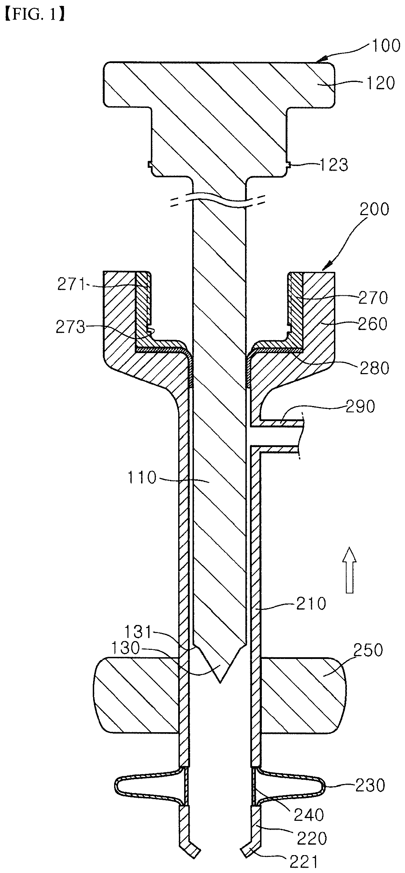

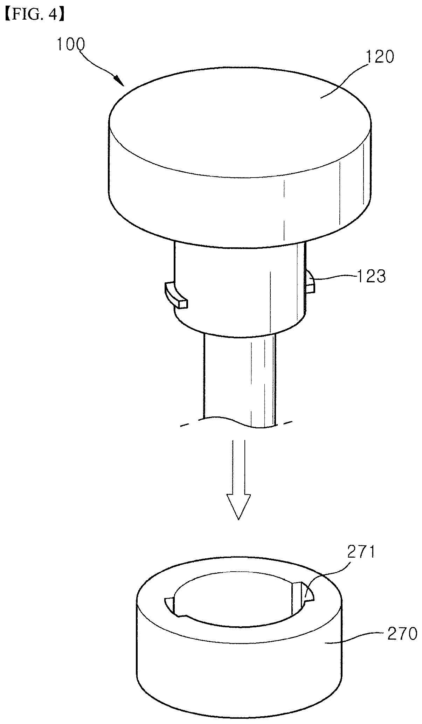

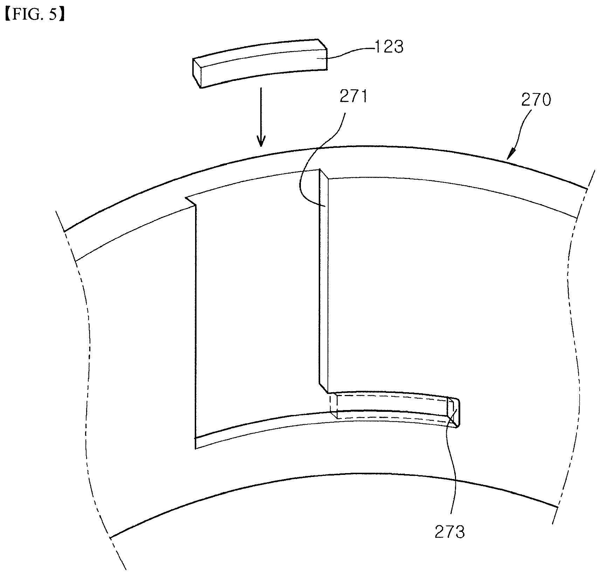

Hereinafter, a surgical trocar according to one embodiment of the present invention will be described with reference to FIG. 1 to FIG. 5.

The surgical trocar includes: a trocar cannula 200 which can be disposed through human skin tissue; and a trocar rod 100 which is inserted into and withdrawn from the trocar cannula 200.

The trocar rod 100 includes a rod body 110, an upper rod head 120 extending upward from the rod body 110, and a lower rod head 130 extending downward from the rod body 110.

The trocar cannula 200 includes a first cannula body 210, a cannula head, a second cannula body 220, a connection unit, a support member 250, a fluid injection portion 290, a sealing member 280, and a locking unit.

The upper rod head 120 corresponds to the cannula head and the lower rod head 130 corresponds to the second cannula body 220.

Specifically, the lower rod head 130 has a stepped head portion 131 stepped with respect to the rod body 110 and is tapered from the stepped head portion 131 to a pointed tip.

The stepped head portion 131 is engaged with a head engaging portion 221 formed at a lower end of the second cannula body 220. That is, when the trocar rod 100 is inserted into the trocar cannula 200, the stepped head portion 131 is engaged with the head engaging portion 221, such that the second cannula body 220 can be moved relative to the first cannula body 210 of the trocar cannula.

The first cannula body 210 has a hollow shape and is provided at an outer surface thereof with the support member 250 and the fluid injection portion 290.

The cannula head includes an outer housing 260 extending from the first cannula body 210 and an inner housing 270 coupled to an inner surface of the outer housing 260. However, it should be understood that the present invention is not limited thereto and the outer housing may be integrally formed with the inner housing.

The outer housing 260 extends upward from the first cannula body 210 and has a larger cross-sectional area than the first cannula body 210.

The inner housing 270 is screwed to the inner surface of the outer housing 260. When the inner housing 270 is coupled to the inner surface of the outer housing 260, a lower surface of the inner housing 270 pushes an edge of the sealing member 280 disposed between the outer housing 260 and the inner housing 270 to secure the sealing member 280.

The second cannula body 220 has a hollow shape and is disposed below the first cannula body 210 to be separated a predetermined distance from the first cannula body 210. The second cannula body 220 includes the head engaging portion 221 formed at the lower end thereof to be bent inward. As described above, the head engaging portion 221 corresponds to the stepped head portion 131.

The second cannula body 220 is vertically moved relative to the first cannula body 210 in the process of inserting the trocar rod 100 into the trocar cannula 200 or withdrawing the trocar rod 100 from the trocar cannula 200.

When the lower portion of the trocar cannula 200 is inserted and placed into the human body, the second cannula body 220 is positioned inside the human body.

The connection unit includes an extension member 230 connecting the first cannula body 210 to the second cannula body 220 and an inner connection member 240 disposed adjacent to and inside the extension member 230 and connecting the first cannula body 210 to the second cannula body 220.

The inner connection member 240 connects the first cannula body 210 to the second cannula body 220 and is formed of a flexible material such that the second cannula body 220 can be vertically moved relative to the first cannula body 210 in the process of inserting the trocar rod 100 into the trocar cannula 200 or withdrawing the trocar rod 100 from the trocar cannula 200.

The first cannula body 210 and the second cannula body 220 may be adhesively bonded to the inner connection member 240 using an adhesive. However, it should be understood that the present invention is not limited thereto and the first cannula body 210 and the second cannula body 220 may be coupled to the inner connection member 240 using a separate fastening member.

When the trocar rod 100 is inserted into the trocar cannula 200 such that the second cannula body 220 is moved relative to the first cannula body 210, the inner connection member 240 is stretched in a longitudinal direction of the first cannula body 210.

In addition, when the trocar rod 100 is withdrawn from the trocar cannula 200, the inner connection member 240 is returned to the original length thereof by elastic restoring force.

The inner connection member 240 is disposed inside the extension member 230 to prevent the extension member 230 from being torn or damaged due to collision with surgical instruments inserted into or withdrawn from the human body through the trocar cannula 200.

However, it should be understood that the present invention is not limited thereto and the inner connection member 240 may be integrally formed with the extension member 230.

The extension member 230 is disposed outside the inner connection member 240 and connects the first cannula body 210 to the second cannula body 220 such that the second cannula body 220 can be vertically moved relative to the first cannula body 210 in the process of inserting the trocar rod 100 into the trocar cannula 200 or withdrawing the trocar rod 100 from the trocar cannula 200.

The extension member 230 may be formed of a shape-memory synthetic resin, such as silicone rubber, which is returned to the original shape thereof when external force is removed.

When not subjected to external force, the extension member 230 is spread in the radial direction of the first cannula body 210 to retain a donut shape.

The extension member 230 may have a constant thickness to exhibit the same strength irrespective of radial location of the extension member 230. Alternatively, the extension member 230 may be thicker and thus have higher strength in a region ranging from an end thereof coupled to each of the first cannula body 210 and the second cannula body 220 to a first radial point than in a region ranging from the first radial point to an outermost radial point so as to remain spread in the radial direction.

However, it should be understood that the present invention is not limited thereto and the extension member 230 may be formed with a vent hole.

As the trocar rod 100 is inserted into the trocar cannula 200 such that the second cannula body 220 is moved relative to the first cannula body 210, the extension member 230 is stretched in the longitudinal direction of the first cannula body 210. That is, when the second cannula body 220 is moved relative to the first cannula body 210, the inner connection member 240 and the extension member 230 are arranged parallel to each other in the longitudinal direction of the first cannula body 210.

After the trocar rod 100 is inserted into the trocar cannula 200 and the extension member 230 is thus stretched in the longitudinal direction of the first cannula body 210, a lower portion of each of the trocar rod 100 and the trocar cannula 200 is inserted into the human body through skin tissue 10.

When the trocar rod 100 is removed after the lower portion of each of the trocar rod 100 and the trocar cannula 200 is inserted into the human body, the extension member 230 is returned to the original shape thereof while being spread in the radial direction of the first cannula body 210.

According to the present invention, an insertion radius of the trocar cannula 200 can be minimized in the process of inserting the lower portion of the trocar cannula 200 into the human body, thereby allowing the human skin tissue 10 to be minimally incised. In addition, after the lower portion of the trocar cannula 200 is inserted into the human body, the extension member 230 is spread in the radial direction of the trocar cannula 200, thereby stably securing the trocar cannula 200 inside the human body.

Further, the extension member 230 can prevent a surface of a surgical instrument or an examination instrument from being stained with blood or water flowing out of the skin tissue 10, thereby preventing contamination of the surgical instrument or the examination instrument while avoiding delay of a surgical operation or an examination operation.

The support member 250 protrudes from an outer peripheral surface of the first cannula body 210 and is located outside the human skin to support the first cannula body 210 when the lower portion of the first cannula body 210 is placed in the human body.

The fluid injection portion 290 communicates with an inner space of the first cannula body 210 and extends radially from the outer surface of the first cannula body 210. The fluid injection portion 290 serves as a flow path through which a gas is injected into the inner space of the first cannula body 210 from the outside. However, it should be understood that the present invention is not limited thereto and the surgical trocar may not include the fluid injection portion 290 when the surgical trocar is being used.

In addition, it should be understood that the present invention is not limited thereto and the first cannula body 210 may be provided on the outer surface thereof with a handle for holding the first cannula body 210.

The sealing member 280 is disposed at an entrance of the cannula head. When the trocar rod 100 is inserted into the trocar cannula 200, the sealing member 280 is opened by the trocar rod 100, and, when the trocar rod 100 is withdrawn from the trocar cannula 200, the sealing member closes the entrance of the cannula head 260.

The inner housing 270 has an annular shape and pushes the edge of the sealing member 280 to secure the sealing member 280 when screwed to the inner surface of the outer housing 260. That is, the edge of the sealing member 280 is disposed between the inner housing 270 and the outer housing 260 to be secured by coupling the inner housing 270 to the outer housing 260.

However, it should be understood that the present invention is not limited thereto and the sealing member 280 may be provided in the form of a one-way check valve inside the cannula head.

According to the present invention, when the trocar rod 100 is withdrawn from the trocar cannula 200 and the sealing member 280 thus closes the entrance of the cannula head 260, a user actuates an external gas supply to supply a gas into the first cannula body 210 through the fluid injection portion 290, such that the gas can be injected into the human body.

The locking unit serves to couple the trocar rod 100 to the trocar cannula 200 when the trocar rod 100 is inserted into the trocar cannula 200 and to release the trocar rod 100 from the trocar cannula 200 when the trocar rod 100 is withdrawn from the trocar cannula 200.

The locking unit includes a coupling protrusion 123 protruding from an outer surface of the upper rod head 120 of the trocar rod 100 and a protrusion coupling portion formed on the cannula head of the trocar cannula 200.

Specifically, the coupling protrusion 123 may include two coupling protrusions symmetrically protruding from a lower outer surface of the upper rod head 120. However, it should be understood that the present invention is not limited thereto and the coupling protrusion 123 may include one or more coupling protrusions and the protrusion coupling portion may correspond in number to the coupling protrusion.

The protrusion coupling portion includes a guide groove 271 for guiding vertical movement of the coupling protrusion 123 and an engaging groove 273 for guiding circumferential movement of the coupling protrusion 123 upon rotation of the trocar rod 100.

The protrusion coupling portion is formed as an L-shaped groove on the inner surface of the inner housing 270. That is, the guide groove 271 is vertically formed on the inner surface of the inner housing 270 and the engaging groove 273 extends from the guide groove 271 and is horizontally formed on the inner surface of the inner housing 270 to be bent in a circumferential direction of the inner housing 270.

Accordingly, as the trocar rod 100 is inserted into the trocar cannula 200, the coupling protrusion 123 is moved along the guide groove 271, and, as the trocar rod 100 is rotated to be coupled to the trocar cannula 200, the coupling protrusion 123 is moved along and seated on the engaging groove 273, such that relative vertical movement of the trocar rod 100 and the trocar cannula 200 can be restricted.

Consequently, the locking unit can keep the extension member 230 stretched in the longitudinal direction of the trocar cannula 200 when a user inserts the trocar rod into the trocar cannula, such that the user can put the surgical trocar into the human body with the extension member stretched in the longitudinal direction of the trocar cannula 200, thereby eliminating a need for the user to continue to hold the trocar rod and the trocar cannula at the same time.

In addition, since the locking unit allows the extension member 230 to remain stretched in the longitudinal direction of the trocar cannula 200, it is possible to solve a problem caused by the extension member 230 which would otherwise be spread in the radial direction of the trocar cannula, that is, a difficulty in putting the surgical trocar into the human body.

Next, a process of disposing the surgical trocar according to the present invention will be described with reference to FIG. 1 to FIG. 5.

First, a user inserts the lower portion of the trocar rod 100 into the trocar cannula 200, as shown in FIG. 1.

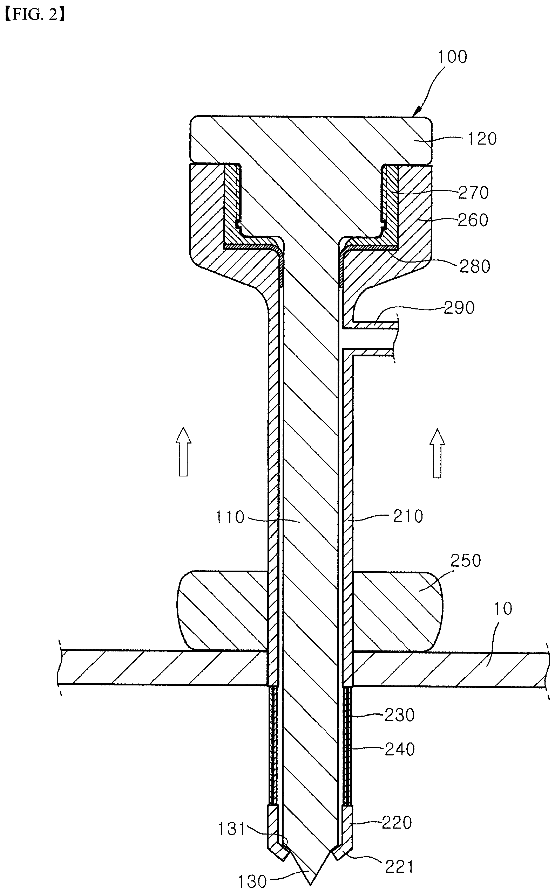

Then, the user pulls the trocar cannula 200 upward such that the tip of the lower rod head 130 is exposed to the outside of the second cannula body 220, as shown in FIG. 2.

Here, the inner connection member 240 and the extension member 230 are stretched in the longitudinal direction of the trocar cannula 200, such that the radius of the extension member 230 is minimized.

Then, the user rotates the trocar rod 100 in a first direction to seat the coupling protrusion 123 of the trocar rod on the engaging groove 273 of the trocar cannula, such the trocar rod 100 and the trocar cannula 200 are secured not to be vertically moved relative to each other with the extension member 230 stretched in the longitudinal direction of the trocar cannula.

Then, the user inserts the lower portion of each of the trocar cannula 200 and the trocar rod 100 into the human body through the human skin tissue 10.

Then, the user rotates the trocar rod 100 in a direction opposite the first direction such that the coupling protrusion 123 of the trocar rod is separated from the engaging groove 273 of the trocar cannula, and then pulls the trocar rod 100 out of the trocar cannula 200.

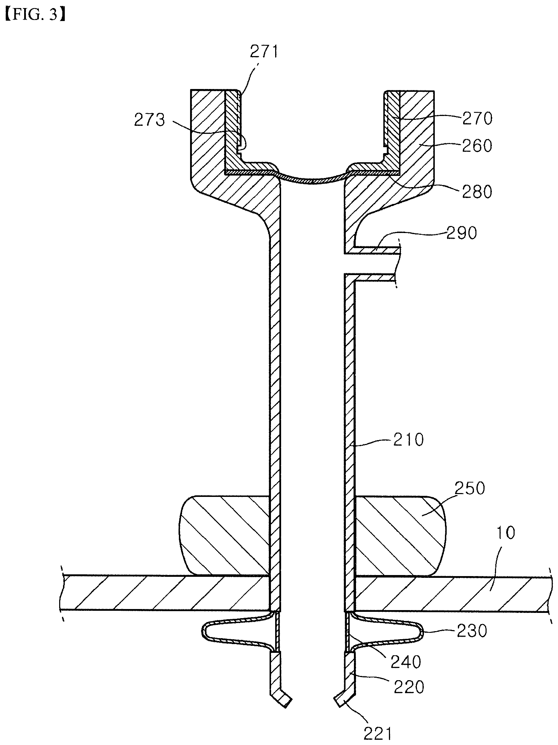

As a result, the extension member 230 is spread in the radial direction of the trocar cannula 200 to be returned to the original shape thereof, that is, the shape thereof before being subjected to external force, as shown in FIG. 3.

Then, the user moves the support member 250 such that the support member 250 is brought into close contact with an outer surface of the skin tissue 10.

As a result, the trocar cannula 200 is supported by both the support member 250 outside the skin tissue 10 and the extension member 230 inside the skin tissue 10 and thus can be stably secured in the human body.

Although some embodiments have been described herein, it should be understood that these embodiments are provided for illustration only and are not to be construed in any way as limiting the present invention, and that various modifications, changes, alterations, and equivalent embodiments can be made by those skilled in the art without departing from the spirit and scope of the invention.

### [Industrial Applicability]

The present invention may be widely used in fields requiring a surgical trocar which has a simple structure and is easy to use while stably supporting a lower portion thereof inside the human body without any additional mechanical equipment, whereby free advance and retreat of surgical instruments can be secured in minimally invasive surgery.

## Claims

1. A surgical trocar comprising:
a trocar cannula capable of being disposed through human skin tissue; and
a trocar rod inserted into and withdrawn from the trocar cannula,
wherein the trocar cannula comprises:
a first cannula body having a hollow shape;
a cannula head disposed at an upper side of the first cannula body;
a second cannula body disposed below the first cannula body to be separated a predetermined distance from the first cannula body; and
a connection unit connecting the first cannula body to the second cannula body such that the second cannula body is vertically moved relative to the first cannula body when the trocar rod is inserted into or withdrawn from the trocar cannula, the connection unit comprising an extension member, the extension member being kept spread in a radial direction of the first cannula body when not subjected to external force and being stretched in a longitudinal direction of the first cannula body when the trocar rod is inserted into the trocar cannula such that the second cannula body is moved relative to the first cannula body.

2. The surgical trocar according to claim 1, wherein the connection unit further comprises: an inner connection member disposed adjacent to and inside the extension member to connect the first cannula body to the second cannula body, the inner connection member being formed of a flexible material such that the second cannula body is vertically moved relative to the first cannula body when the trocar rod is inserted into or withdrawn from the trocar cannula.

3. The surgical trocar according to claim 1, wherein the trocar rod comprises:
a rod body;
an upper rod head extending upward from the rod body; and
a lower rod head extending downward from the rod body, the lower rod head having a stepped head portion, and
wherein the second cannula body has a head engaging portion formed at a lower end thereof, the head engaging portion being configured to be engaged with the stepped head portion.

4. The surgical trocar according to any one of claims 1 to 3, wherein the extension member is formed of a shape-memory synthetic resin such that a lower portion of each of the trocar rod and the trocar cannula can be inserted into a human body with the extension member stretched in the longitudinal direction of the first cannula body and the extension member can be spread in the radial direction of the first cannula body to be returned to an original shape thereof when the trocar rod is removed after the lower portion of each of the trocar rod and the trocar cannula is inserted into the human body.

5. The surgical trocar according to any one of claims 1 to 3, further comprising:
a locking unit,
wherein the locking unit couples the trocar rod to the trocar cannula when the trocar rod is inserted into the trocar cannula and releases the trocar rod from the trocar cannula when the trocar rod is withdrawn from the trocar cannula.

6. The surgical trocar according to claim 5, wherein the locking unit comprises:
a coupling protrusion protruding from an outer surface of the upper rod head of the trocar rod; and
a protrusion coupling portion formed at the cannula head of the trocar cannula.

7. The surgical trocar according to claim 6, wherein the cannula head comprises: an outer housing extending from the first cannula body; and an inner housing coupled to an inner surface of the outer housing, and the protrusion coupling portion is formed on an inner surface of the inner housing.

8. The surgical trocar according to claim 7, wherein the protrusion coupling portion comprises:
a guide groove formed on the inner surface of the inner housing to guide vertical movement of the coupling protrusion; and
an engaging groove extending from the guide groove, the engaging groove being bent in a circumferential direction of the inner housing to guide movement of the coupling protrusion upon rotation of the trocar rod.

9. The surgical trocar according to claim 7, further comprising:
a sealing member disposed at an entrance of the cannula head,
wherein the sealing member is opened when the trocar rod is inserted into the trocar cannula and closes the entrance of the cannula head when the trocar rod is withdrawn from the trocar cannula.

10. The surgical trocar according to claim 9, wherein, when the inner housing is coupled to the inner surface of the outer housing, a lower surface of the inner housing pushes an edge of the sealing member disposed between the outer housing and the inner housing to secure the sealing member.
